# EUROPEAN PATENT SPECIFICATION

(11) **EP 3 003 384 B1**
(45) Date of publication and mention of the grant of the patent: **26.10.2016**
(21) Application number: 14726544.1
(22) Date of filing: 02.05.2014
(51) Int. Cl.: A61K 47/20, A61K 47/22, A61K 47/00, A61K 47/26, A61K 31/138, A61K 9/00

(54) **ORAL SOLUTION COMPRISING ATOMOXETINE HYDROCHLORIDE**
ORALE LÖSUNG MIT ATOMOXETIN-HYDROCHLORID
SOLUTION ORALE CONTENANT DU CHLORHYDRATE D'ATOMOXÉTINE

(30) Priority: 24.03.2014 GR 20140100157
(43) Date of publication of application: 13.04.2016
(73) Proprietor: Lamda Laboratories S.A., Attiki (GR)
(72) Inventor: KARATZAS, Angelos, GR-15235 Vrilissia Attica (GR); STAPPA, Argyro, GR-15231 Chalandri Attica (GR); KOTSIANIS, Ilias, GR-14342 Nea Filadelfeia Attica (GR); APOSTOLOU, Konstantinos, GR-15344 Gerakas Attica (GR)
(74) Representative: Roukounas, Dimitrios
(86) International application number: PCT/EP2014/059020
(87) International publication number: WO 2015/144255

(56) References cited:
- WO-A2-2006/020348
- US-A1- 2005 152 974
- US-A1- 2011 076 239

## Description

### TECHNICAL FIELD

The present invention refers to palatable oral pharmaceutical solutions comprising high concentrations of atomoxetine hydrochloride.

### BACKGROUND OF THE INVENTION

Atomoxetine (Formula I) is a highly selective and potent inhibitor of the pre-synaptic noradrenaline transporter, its presumed mechanism of action, without directly affecting the serotonin or dopamine transporters and it is 2 and 9 times more effective than the racemic mixture and the (+)-enantiomer, respectively, of N-methyl-3-(2-methylphenoxy)-3-phenylpropylamine (tomoxetine, "TMX"), disclosed in EP0052492. Atomoxetine is the (R)-(-) enantiomer of Tomoxetine.

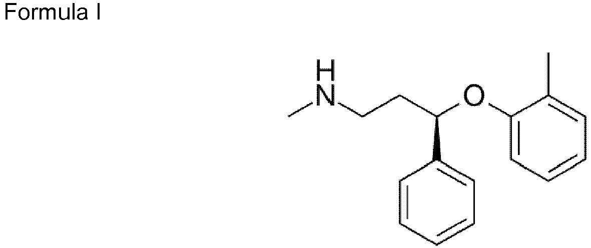

The hydrogen chloride salt of atomoxetine, atomoxetine HCl, is marketed as Strattera®, which is prescribed as oral capsules having dosages of 10 mg, 18 mg, 25 mg, 40 mg, and 60 mg for the treatment of attention deficit hyperactivity disorder (ADHD), in children over 6 years of age and adults.

Although oral solid dosage forms such as capsules are very popular for reasons that are mainly due to ease of management, for certain users (e.g. children and the elderly) these forms are not necessarily a convenient option, especially due to difficulty in swallowing these forms. This lack of convenience results in high incidence of non-compliance and ineffective therapy.

Moreover, the Patient Information Leaflet (PIL) of Strattera® capsules discloses a dosing scheme for children over 6 years of age and adolescents up to 70 kg according to which dosing is adjusted to their body weight whereas the corresponding adult-dosing is subjected to titration dependent on their response. The concept of tailored treatment sets as prerequisite pharmaceutical forms, such as oral solutions, that enable dose fractioning.

An atomoxetine oral solution (4 milligrams per milliliter [mg/mL]) is known to have been in the subject of a Bioequivalence study in healthy adult male Japanese subjects, organized by Eli Lilly in 2010.

However, as a means of both ensuring flexibility in dose titration and advanced patient compliance, the development of oral pharmaceutical solutions with higher concentrations of atomoxetine hydrochloride which can allow smaller dose is definitely an existing need.

Although atomoxetine hydrochloride is soluble in water (27.8 mg/ml at room temperature) the desire for the development of a high concentrated oral solution dosage form of atomoxetine hydrochloride is complicated by the fact that it is extremely bitter. According to "Huda. I. G et al., Taste masked chewable dispersible tablet of atomoxetine HCl, International Journal of Pharmacy and Pharmaceutical Sciences, Vol 5, Suppl 1, 2013" as well as to "Y. Deepthi Priya et al., An approach for taste masking of bitter drug atomoxetine HCl, International Journal of Advances in Pharmaceutical Research, Vol. 2, Issue. 4/119-121, April 2011" atomoxetine hydrochloride is an intensely bitter drug.

Thus, it is challenging to formulate an atomoxetine hydrochloride solution especially at high concentrations since it is generally considered difficult to effectively taste mask the bitterness in concentrated liquid dosage forms.

A technically bigger challenge is the development of paediatric formulations. High concentrations of taste masking agents such as sweeteners, sugar alcohols and/or flavors may be necessary for masking the taste of highly bitter drugs but such high quantities may decrease sharply the solubility of even readily soluble substances. The problem is bigger due to the fact that other solvents such as alcohols, polyethylene glycol and propylene glycol are typically avoided in pediatric formulations because of their toxicity especially to the very young.

The present invention addresses the problems of the prior art knowledge by advantageously providing palatable high concentrated atomoxetine hydrochloride oral pharmaceutical solutions.

### SUMMARY OF THE INVENTION

The present invention is directed to oral pharmaceutical solutions comprising high concentrations of atomoxetine hydrochloride in association with a pharmaceutically acceptable liquid carrier.

The present invention provides a pharmaceutical composition comprising atomoxetine hydrochloride as an active ingredient and a liquid carrier comprising a sweetener which is selected from the group consisting of sucralose, saccharin, sodium, potassium or calcium saccharin, aspartame, stevioside and rebaudioside, along with a combination of two sugar alcohols, namely xylitol and maltitol. The formulations of the invention provide a synergistic taste masking effect. The term "synergistic taste masking effect" means a taste masking effect that is not additive, as would be predicted from the individual effect of each excipient, but which instead gives a level of taste masking above that which would be predicted, i.e., is synergistic.

The bitterness value of both atomoxetine hydrochloride and formulations comprising atomoxetine hydrochloride is measured following the guidelines of European Pharmacopoeia (Ph. Eur. 8.0 - paragraph 2.8.15).

The oral pharmaceutical solutions of atomoxetine hydrochloride according to the invention present excellent taste masking effect while at the same time allow the optimal selection of concentration of taste masking excipients at the lowest feasible level.

The oral pharmaceutical solutions of atomoxetine hydrochloride according to the invention may be free of additional polyhydric and sugar alcohols commonly used for the taste masking of oral liquid pharmaceutical compositions, such as glycerol, sorbitol and mannitol, which may raise additional safety and toxicity issues. Absence of additional polyhydric and sugar alcohols is important particularly in case of paediatric formulations.

The oral pharmaceutical solutions of atomoxetine hydrochloride according to the invention may also optionally contain additional ingredients commonly used in the preparation of oral liquid pharmaceutical compositions, such as antimicrobial preservatives, antioxidants, viscosity adjusting agents, flavoring agents and the like.

### DETAILED DESCRIPTION OF THE INVENTION

The present invention provides atomoxetine hydrochloride oral pharmaceutical solutions.

The inventors' early research confirmed that atomoxetine hydrochloride is an intensely bitter drug by calculating a bitterness value of much more than 100,000, employing an adult sensory panel of 8 persons and following the guidelines described in Ph. Eur. 8.0 - paragraph 2.8.15.

Moreover, the inventors' early research for taste masking of atomoxetine hydrochloride solution in water utilizing certain taste masking excipients such as ethanol and mannitol, proved fruitless in that the taste imparted by these agents was additive to the already bad taste of atomoxetine hydrochloride.

The technically bigger challenge was the efficient use of other taste masking agents such as sweeteners, sugar alcohols and/or flavors, since the addition of high quantities of them in aqueous atomoxetine hydrochloride solutions proved to decrease sharply the solubility of the active ingredient.

However, it has been surprisingly found that certain sugar alcohols, namely, xylitol and maltitol provide a synergistic taste masking effect that could not be predicted from the taste masking effect of xylitol and maltitol as individual agents.

Moreover, it has been surprisingly revealed that the combination of certain sweeteners along with the combination of xylitol and maltitol provides a palatable atomoxetine hydrochloride pharmaceutical oral solution.

The oral pharmaceutical solutions of the present invention comprise from 6 mg/ml to 10 mg/ml of atomoxetine hydrochloride, one or more sweeteners selected from the group consisting of sucralose, saccharin, sodium, potassium or calcium saccharin, aspartame, stevioside and rebaudioside wherein the total concentration of sweetener is from 5 mg/ml to 11 mg/ml, from 170 mg/ml to 210 mg/ml of xylitol, from 130 mg/ml to 170 mg/ml of maltitol, and water, wherein the pH of the solution is from 3.5 to 4.5.

Preferably, the concentration of atomoxetine hydrochloride in the compositions of the present invention is from 7 mg/ml to 9 mg/ml.

Preferably, the concentration of xylitol in the compositions of the present invention is from 180 mg/ml to 200 mg/ml.

Preferably, the concentration of maltitol in the compositions of the present invention is from 140 mg/ml to 160 mg/ml.

Preferably, the total concentration of sweetener in the compositions of the present invention is from 6 to 10 mg/ml.

Preferably, the oral pharmaceutical solutions of the present invention comprise one or more sweeteners selected from the group consisting of sucralose, saccharin, sodium or calcium saccharin, stevioside and rebaudioside. More preferably, the oral pharmaceutical solutions of the present invention comprise one or more sweeteners selected from the group consisting of sucralose and sodium saccharin. Even more preferably, the oral pharmaceutical solutions of the present invention comprise as sweetener sucralose.

Any suitable system which acts as a buffer in the pH region around 4 can be used in the compositions of the present invention. Such buffer systems include phosphate buffers such as sodium and potassium monobasic phosphate, sodium, potassium and calcium citrate buffers, as well as other pharmaceutically acceptable buffers.

Buffer solutions work effectively if they have sufficient buffering capacity to resist the change in pH expected during the production or storage period. Buffer solutions when used in the compositions of the present invention should be prepared with at least a minimum buffer capacity, so as to achieve pH values of the finished oral solution in the range from 3.5 to 4.5.

The compositions of the present invention exhibit excellent taste masking effect even when they do not contain additional taste masking agents, such as ethanol, glycerol, sorbitol and mannitol. The absence of these agents is very important, particularly in the case of formulations intended for children.

The oral pharmaceutical solutions of atomoxetine hydrochloride, according to the invention may also optionally contain additional excipients commonly used in preparing oral liquid compositions, such as antimicrobial preservatives, antioxidants, viscosity adjusting agents, and flavouring agents.

Examples of preservatives that may be used in the present invention are methyl-p-hydroxybenzoate, propyl-p-hydroxybenzoate, ethyl-p-hydroxybenzoate and their sodium salts, benzoic acid, sodium benzoate and any combination thereof.

The antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate and any combination thereof.

The viscosity adjusting agents may be, for example, sodium carboxymethylcellulose, methylcellulose, hydroxypropylcellulose, polyvinylpyrrolidone or any combination thereof.

Appropriate flavouring agents may include any of the many non-toxic natural or artificial flavouring agents known in the daily practice. In particular, the flavouring agents used may include one or more of a variety of natural or artificial fruit flavours. Alternatively, or in addition, the flavouring agents may include one or more of natural or artificial vanilla, chocolate, and caramel flavourings, amongst others.

The compositions of the present invention may be prepared using methods well-known in the prior art. For example they may be prepared using the following process;
- Purified water is placed into a main vessel equipped with a stirrer.
- Atomoxetine hydrochloride is added and mixed until complete dissolution.
- The preservative, if present, is added to the same vessel and mixed until complete dissolution.
- Xylitol is added and mixed until complete dissolution.
- The solution is heated until it reaches the temperature of 30 °C and maltitol is added and mixed until complete dissolution.
- A buffer solution is prepared in an auxiliary vessel by adding a buffering agent in water and stirring until it is completely dissolved. The buffer solution is transferred to the main vessel and mixed.
- The sweetener and flavor, if present, are added and mixed until they are completely dissolved.
- The pH of the oral solution is adjusted to the desired value, if needed.
- The volume of the oral solution is adjusted to the desired batch volume by adding buffer solution.

### EXAMPLES

The following examples show the influence of the proposed, according to the invention, carrier, on the palatability and solubility of atomoxetine hydrochloride.

### EXAMPLE 1

Five liquid compositions were studied in relation to their taste and appearance. The compositions were subjected to sensory evaluation by a panel of four members with respect to bitter taste. The evaluation was performed by classifying bitter tastes into the following three classes:
Level 3: Strong bitter taste is sensed - Level 2: Some bitter taste is sensed - Level 1: No bitter taste is sensed.

These compositions were prepared through a manufacturing process, where a buffer solution is prepared and adjusted to the desired pH value. Buffer corresponding to approximately 30% of the final batch size is added to a mixing vessel (main mixing vessel). The active ingredient is added to the above solution under continuous stirring until it is completely dissolved. The sweetener is then added to the above solution under continuous stirring. The pH of the solution is adjusted, if necessary, to the desired region by adding aqueous sodium hydroxide or hydrochloric acid solution. The volume is adjusted to the desired batch volume by adding buffer solution.

**Table 1**

| | **Composition** | | | | |
|---|---|---|---|---|---|
| | **A1** | **A2** | **A3** | **A4** | **A5** |
| **Active ingredient** | mg/ml | | | | |
| Atomoxetine hydrochloride | 2.28 | 4.57 | 6.84 | 9.10 | 11.4 |

| **Excipients** | | | | | |
|---|---|---|---|---|---|
| Sodium saccharin | 5 | 5 | 5 | 5 | 5 |
| Sorbitol | 150 | 150 | 150 | 150 | 150 |
| Glycerol | 150 | 150 | 150 | 150 | 150 |
| Pharmaceutically acceptable buffer | q.s. 1ml | q.s. 1ml | q.s. 1ml | q.s. 1ml | q.s. 1ml |
| pH of the oral solution | 4.0 | 4.0 | 4.0 | 4.0 | 4.0 |
| | | | | | |
| **Taste of solution** | Palatable (level 1) | Palatable (level 1) | Bitter (level 3) | Bitter (level 3) | Bitter (level 3) |
| **Appearance of solution** | clear | clear | clear | clear | white precipitate |

As revealed, when the concentration of atomoxetine hydrochloride is increased beyond 4 mg/ml, the oral solution becomes increasingly problematic to readily taste-masking with the addition of sweeteners.

### EXAMPLE 2

Eighteen liquid compositions were studied in relation to their appearance and bitterness value according to the method described in paragraph 2.8.15 of Ph. Eur. - 8.0. An adult sensory panel comprising of 8 persons was employed.

These compositions were prepared through a manufacturing process, where purified water is placed into a main vessel equipped with a stirrer. Atomoxetine hydrochloride is added and mixed until complete dissolution. Sodium benzoate is added to the same vessel and mixed until complete dissolution. Xylitol is added and mixed until complete dissolution. The solution is heated until it reaches the temperature of 30 °C and maltitol is added and mixed until complete dissolution. A buffer solution is prepared in an auxiliary vessel by adding sodium dihydrogen phosphate anhydrous and stirring until it is completely dissolved. The solution is transferred to the main vessel and mixed. The sweetener and flavor are added and mixed until they are completely dissolved. The pH of the oral solution is adjusted to the desired value, if needed, by adding 10% phosphoric acid aqueous solution and/or sodium hydroxide 1N aqueous solution. The volume of the oral solution is adjusted to the desired batch volume by adding buffer solution.

**Table 2:**

| | **Compositions (mg/ml)** | | | | | | |
|---|---|---|---|---|---|---|---|
| **Ingredient** | **B1** | **B2** | **B3** | **B4** | **B5** | **B6** | **B7** |
| Atomoxetine HCl | 9.14 | 9.14 | 9.14 | 9.14 | 9.14 | 9.14 | 9.14 |
| Sucralose | 10 | 10 | 10 | 10 | 8 | 8 | 8 |
| Sodium benzoate | 0.8 | 0.8 | 0.8 | 0.8 | 0.8 | 0.8 | 0.8 |
| Sorbitol | 300 | - | 200 | 100 | - | - | - |
| Glycerol | - | 300 | 100 | 200 | - | - | - |
| Xylitol | - | - | - | - | 350 | - | 200 |
| Maltitol | - | - | - | - | - | 350 | 150 |
| Blackberry flavor | 17 | 17 | 17 | 17 | 17 | 17 | 17 |
| NaOH 1.0N or 10% phosphoric acid solution | pH 5.5 | | | | | | |
| Pharmaceutically acceptable buffer solution | q.s to 1.0 mL | | | | | | |
| | | | | | | | |
| Taste of solution (Bitterness value) | bitter (22546) | bitter (23459) | bitter (20101) | bitter (19632) | bitter (4973) | bitter (6209) | palatable (2373) |
| Solubility (appearance) | white precipitate | clear | white precipitate | clear | white precipitate | white precipitate | white precipitate |

As revealed, at pH 5.5, when the concentration of xylitol and maltitol is high enough to effectively taste-mask the solution, a precipitate of the active ingredient is formed. Loss of atomoxetine hydrochloride was indicated by a decrease in its concentration (measured by HPLC) in the supernatant solution.

**Table 3:**

| | **Compositions (mg/ml)** | | | | | |
|---|---|---|---|---|---|---|
| **Ingredient** | **B8** | **B9** | **B10** | **B11** | **B12** | **B13** |
| Atomoxetine HCl | 9.14 | 9.14 | 9.14 | 9.14 | 9.14 | 9.14 |
| Sucralose | 10 | 10 | 10 | 10 | 10 | 8 |
| Sodium benzoate | 0.8 | 0.8 | 0.8 | 0.8 | 0.8 | 0.8 |
| Sorbitol | - | 100 | - | - | - | - |
| Glycerol | 300 | 200 | 200 | 100 | - | - |
| Xylitol | - | - | 100 | - | 100 | 100 |
| Maltitol | - | - | - | 200 | 200 | 200 |
| Blackberry flavor | 17 | 17 | 17 | 17 | 17 | 17 |
| NaOH 1.0N or 10% phosphoric acid solution | pH 4.0 | | | | | |
| Pharmaceutically acceptable buffer solution | q.s to 1.0 mL | | | | | |
| | | | | | | |
| Taste of solution (Bitterness value) | bitter (23903) | bitter (19903) | bitter (15003) | bitter (11209) | bitter (3773) | bitter (5312) |
| Solubility (appearance) | clear | clear | clear | clear | clear | clear |

Table 3 shows compositions falling outside the scope of the present invention. It is clear that at pH 4, the combination of sweetener with polyhydric or sugar alcohols, even with xylitol and maltitol outside the claimed range, does not produce satisfactory results.

**Table 4:**

| | **Compositions (mg/ml)** | | | | |
|---|---|---|---|---|---|
| **Ingredient** | **B14** | **B15** | **B16** | **B17** | **B18** |
| Atomoxetine HCl | 9.14 | 9.14 | 9.14 | 9.14 | 9.14 |
| Sucralose | 8 | 8 | 12 | 8 | 8 |
| Sodium benzoate | 0.8 | 0.8 | 0.8 | 0.8 | 0.8 |
| Xylitol | 350 | - | 200 | 200 | 200 |
| Maltitol | - | 350 | 160 | 160 | 160 |
| Blackberry flavor | 17 | 17 | 17 | 17 | 12 |
| NaOH 1.0N or 10% phosphoric acid solution | pH 4.0 | | | | |
| Pharmaceutically acceptable buffer solution | q.s to 1.0 mL | | | | |
| | | | | | |
| Taste of solution (Bitterness value) | bitter (5102) | bitter (6390) | palatable (773) | palatable (1673) | palatable (1904) |
| Solubility (appearance) | clear | clear | white precipitate | clear | clear |

Table 4 shows the unexpected result that the actual taste masking effect of the combination of xylitol and maltitol is not additive but synergistic. However, in order to obtain satisfactory results the concentrations of the sweetener, xylitol and maltitol as well as the pH of the solution have to be within certain ranges.

### EXAMPLE 3

Six liquid compositions were studied in relation to their appearance and bitterness value according to the method described in paragraph 2.8.15 of Ph. Eur. - 8.0. An adult sensory panel comprising of 8 persons was employed.

These compositions were prepared through a manufacturing process, where purified water is placed into a main vessel equipped with a stirrer. Atomoxetine hydrochloride is added and mixed until complete dissolution. Sodium benzoate is added to the same vessel and mixed until complete dissolution. Xylitol is added and mixed until complete dissolution. The solution is heated until it reaches the temperature of 30 °C and maltitol is added and mixed until complete dissolution. A buffer solution is prepared in an auxiliary vessel by adding sodium dihydrogen phosphate anhydrous and stirring until it is completely dissolved. The solution is transferred to the main vessel and mixed. The sweetener and flavor are added and mixed until they are completely dissolved. The pH of the oral solution is adjusted to the desired value, if needed, by adding 10% phosphoric acid aqueous solution and/or sodium hydroxide 1N aqueous solution. The volume of the oral solution is adjusted to the desired batch volume by adding buffer solution.

**Table 5:**

| | **Compositions (mg/ml)** | | | | | |
|---|---|---|---|---|---|---|
| **Ingredient** | **C1** | **C2** | **C3** | **C4** | **C5** | **C6** |
| Atomoxetine HCl | 9.14 | 9.14 | 9.14 | 9.14 | 9.14 | 9.14 |
| Sucralose | 8 | - | 5 | 8 | 4 | 8 |
| Sodium saccharin | - | 8 | - | - | 4 | - |
| Sodium Benzoate | 0.8 | 0.8 | 0.8 | 0.8 | 0.8 | 0.8 |
| NaOH 1.0N or 10% phosphoric acid solution | pH 4.0 | | | | | |
| Xylitol | 180 | 180 | 200 | 150 | 160 | 200 |
| Maltitol | 140 | 140 | 160 | 120 | 200 | 200 |
| Blackberry flavor | 12 | 12 | 12 | 12 | 12 | 12 |
| Pharmaceutically acceptable buffer solution | q.s to 1.0 mL | | | | | |
| | | | | | | |
| Taste of solution (Bitterness value) | palatable (1673) | palatable (2173) | palatable (2904) | bitter (5973) | bitter (4904) | palatable (974) |
| Appearance of solution | clear | clear | clear | clear | clear | white precipitate |

As revealed, when the pH is at 4.0 and the total concentration of sugar alcohols (xylitol, maltitol) is around 350 mg/ml then the concentration of the sweetener for effectively taste-masking atomoxetine hydrochloride solutions should be at least 5 mg/ml.

Overall, it is revealed that intermediate concentration ranges of sweetener, along with a combination of maltitol and xylitol, in specific concentration ranges, exert optimum taste masking effect without compromising the solubility of atomoxetine hydrochloride in the liquid compositions.

## Claims

1. Oral pharmaceutical solution comprising from 6 mg/ml to 10 mg/ml of atomoxetine hydrochloride, one or more sweeteners selected from the group consisting of sucralose, saccharin, sodium, potassium or calcium saccharin, aspartame, stevioside and rebaudioside, wherein the total concentration of sweetener is from 5 mg/ml to 11 mg/ml, from 170 mg/ml to 210 mg/ml of xylitol, from 130 mg/ml to 170 mg/ml of maltitol, and water, wherein the pH of the solution is from 3.5 to 4.5.

2. Oral pharmaceutical solution according to claim 1, wherein the concentration of atomoxetine hydrochloride is from 7 mg/ml to 9 mg/ml.

3. Oral pharmaceutical solution according to claim 1 or 2, wherein the concentration of xylitol is from 180 mg/ml to 200 mg/ml.

4. Oral pharmaceutical solution according to any one of claims 1 to 3, wherein the concentration of maltitol is from 140 mg/ml to 160 mg/ml.

5. Oral pharmaceutical solution according to any one of claims 1 to 4, wherein the total concentration of sweetener is from 6 to 10 mg/ml.

6. Oral pharmaceutical solution according to any one of claims 1 to 5, wherein the sweetener is selected from the group consisting of sucralose, saccharin, sodium or calcium saccharin, stevioside and rebaudioside.

7. Oral pharmaceutical solution according to any one of claims 1 to 6, wherein the sweetener is selected from the group consisting of sucralose and sodium saccharin.

8. Oral aqueous pharmaceutical solution according to any one of claims 1 to 7, wherein the sweetener is sucralose.

9. Oral aqueous pharmaceutical solution according to any one of claims 1 to 8, which is free of ethanol, sorbitol, glycerol and mannitol.

10. Oral aqueous pharmaceutical solution according to any one of claims 1 to 9, which consists of
9.14 mg/ml atomoxetine hydrochloride,
8 mg/ml sucralose,
200 mg/ml xylitol,
160 mg/ml maltitol,
0.8 mg/ml sodium benzoate,
17 mg/ml blackberry flavor,
and a pharmaceutically acceptable buffer solution, wherein the pH of the oral solution is 4.0.

## Patentansprüche

1. Orale pharmazeutische Lösung, umfassend von 6 mg/ml bis 10 mg/ml Atomoxetin-Hydrochlorid, ein oder mehrere Süßungsmittel, ausgewählt aus der Gruppe bestehend aus Sucralose, Saccharin, Natrium-, Kalium- oder Calciumsaccharin, Aspartam, Steviosid und Rebaudiosid, wobei die Gesamtkonzentration des Süßungsmittels von 5 mg/ml bis 11 mg/ml beträgt, von 170 mg/ml bis 210 mg/ml Xylit, von 130 mg/ml bis 170 mg/ml Maltit und Wasser, wobei der pH der Lösung von 3,5 bis 4,5 beträgt.

2. Orale pharmazeutische Lösung nach Anspruch 1, wobei die Konzentration von Atomoxetin-Hydrochlorid von 7 mg/ml bis 9 mg/ml beträgt.

3. Orale pharmazeutische Lösung nach Anspruch 1 oder 2, wobei die Konzentration von Xylit von 180 mg/ml bis 200 mg/ml beträgt.

4. Orale pharmazeutische Lösung nach einem der Ansprüche 1 bis 3, wobei die Konzentration von Maltit von 140 mg/ml bis 160 mg/ml beträgt.

5. Orale pharmazeutische Lösung nach einem der Ansprüche 1 bis 4, wobei die Gesamtkonzentration des Süßungsmittels von 6 bis 10 mg/ml beträgt.

6. Orale pharmazeutische Lösung nach einem der Ansprüche 1 bis 5, wobei das Süßungsmittel aus der Gruppe ausgewählt ist, die aus Sucralose, Saccharin, Natrium- oder Calciumsaccharin, Steviosid und Rebaudiosid besteht.

7. Orale pharmazeutische Lösung nach einem der Ansprüche 1 bis 6, wobei das Süßungsmittel aus der Gruppe ausgewählt ist, die aus Sucralose und Natriumsaccharin besteht.

8. Orale wässrige pharmazeutische Lösung nach einem der Ansprüche 1 bis 7, wobei das Süßungsmittel Sucralose ist.

9. Orale wässrige pharmazeutische Lösung nach einem der Ansprüche 1 bis 8, die frei von Ethanol, Sorbitol, Glycerin und Mannitol ist.

10. Orale wässrige pharmazeutische Lösung nach einem der Ansprüche 1 bis 9, die aus
9,14 mg/ml Atomoxetin-Hydrochlorid,
8 mg/ml Sucralose,
200 mg/ml Xylit,
160 mg/ml Maltit,
0,8 mg/ml Natriumbenzoat,
17 mg/ml Brombeeraroma
und einer pharmazeutisch akzeptablen Pufferlösung besteht, wobei der pH der oralen Lösung 4,0 beträgt.

## Revendications

1. Solution pharmaceutique orale comprenant de 6 mg/mL à 10 mg/mL de chlorhydrate d'atomoxétine, un ou plusieurs édulcorants sélectionnés dans le groupe constitué du sucralose, de la saccharine, de la saccharine de sodium, de potassium ou de calcium, de l'aspartame, du stévioside et du rébaudioside, dans laquelle la concentration totale en édulcorant est de 5 mg/mL à 11 mg/mL, de 170 mg/mL à 210 mg/mL de xylitol, de 130 mg/mL à 170 mg/mL de maltitol, et de l'eau, dans laquelle le pH de la solution est de 3,5 à 4,5.

2. Solution pharmaceutique orale selon la revendication 1, dans laquelle la concentration en chlorhydrate d'atomoxétine est de 7 mg/mL à 9 mg/mL.

3. Solution pharmaceutique orale selon la revendication 1 ou 2, dans laquelle la concentration en xylitol est de 180 mg/mL à 200 mg/mL.

4. Solution pharmaceutique orale selon l'une quelconque des revendications 1 à 3, dans laquelle la concentration en maltitol est de 140 mg/mL à 160 mg/mL.

5. Solution pharmaceutique orale selon l'une quelconque des revendications 1 à 4, dans laquelle la concentration totale en édulcorant est de 6 à 10 mg/mL.

6. Solution pharmaceutique orale selon l'une quelconque des revendications 1 à 5, dans laquelle l'édulcorant est sélectionné dans le groupe constitué du sucralose, de la saccharine, de la saccharine de sodium ou de calcium, du stévioside et du rébaudioside.

7. Solution pharmaceutique orale selon l'une quelconque des revendications 1 à 6, dans laquelle l'édulcorant est sélectionné dans le groupe constitué du sucralose et de la saccharine sodique.

8. Solution pharmaceutique orale aqueuse selon l'une quelconque des revendications 1 à 7, dans laquelle l'édulcorant est le sucralose.

9. Solution pharmaceutique orale aqueuse selon l'une quelconque des revendications 1 à 8, qui est dépourvue d'éthanol, de sorbitol, de glycérol et de mannitol.

10. Solution pharmaceutique orale aqueuse selon l'une quelconque des revendications 1 à 9, qui est constituée de :
9,14 mg/mL de chlorhydrate d'atomoxétine,
8 mg/mL de sucralose,
200 mg/mL de xylitol,
160 mg/mL de maltitol,
0,8 mg/mL de benzoate de sodium,
17 mg/mL d'arôme de mûre,
et d'une solution tampon pharmaceutiquement acceptable, dans laquelle le pH de la solution orale est de 4,0.
